# EUROPEAN PATENT SPECIFICATION

(11) **EP 3 377 126 B1**
(45) Date of publication and mention of the grant of the patent: **22.05.2019**
(21) Application number: 17761928.5
(22) Date of filing: 26.07.2017
(51) Int. Cl.: A61L 27/44, A61L 27/46

(54) **THREADS OF CROSS-LINKED HYALURONIC ACID AND HYDROXYAPATITE**
STRÄNGE AUS VERNETZTER HYALURONSÄURE UND HYDROXYLAPATIT
FILS D'ACIDE HYALURONIQUE RÉTICULÉ ET D'HYDROXYAPATITE

(30) Priority: 27.07.2016 US 201662367137 P
(43) Date of publication of application: 26.09.2018
(73) Proprietor: Marbelle Threads Ltd., 7120101 Lod (IL)
(72) Inventor: SEGAL, David Dadi, 6436805 Tel-Aviv (IL); GOLDBERG, Eran, 5259321 Ramat Gan (IL); SHKLANOVSKY, Lital, 5540101 Kiryat Ono (IL); COHEN, Aviv, 5826709 Holon (IL); GOLDSHAID-ZMIRI, Liat, 4976001 Petach Tikva (IL)
(74) Representative: Russell, Tim
(86) International application number: PCT/IL2017/050837
(87) International publication number: WO 2018/020501

(56) References cited:
- WO-A1-2011/109129
- WO-A1-2013/053457
- WO-A1-2014/165113
- US-A1- 2015 257 989

## Description

### Field of the invention

The present invention relates to methods of manufacturing of composite material threads comprising cross-linked hyaluronic acid and hydroxyapatite, to the threads themselves, and use thereof in cosmetic applications and in medical applications.

### Background of the invention

Hyaluronic acid is a common component of cosmetic preparations and is used in several cosmetic procedures, particularly in filling wrinkles. Natural hyaluronic acid has poor *in-vivo* stability due to rapid enzymatic degradation and hydrolysis and, accordingly, various chemically modified forms of hyaluronic acid (e.g., cross-linked forms, ionically modified forms, esterified forms, etc.) have been prepared to address poor stability. For example, PCT patent application WO2013053457 discloses a composition of hydroxyapatite and two hyaluronic acid polymers of different molecular weight cross-linked together. Additionally, US patent applications publication Nos. 20150257989 and 2015238525 describe cohesive cross-linked hyaluronic acid gel filled with hydroxyapatite particles.

Hydroxyapatite has a chemical composition which is very similar to that of the mineral phase of bone. Its biological properties and its biocompatibility make it an excellent bone-substitute product. Bone colonization by the substitute is usually highly dependent upon the porous characteristics of the material and in particular on pore size and distribution, and the interconnection between macropores (number and size). The interconnections are tunnels that allow the passage of cells and the circulation of blood between the pores and thus promote bone formation within the substitute. Calcium hydroxyapatite (CaHAp) is a mineral species of the phosphate family, having the formula Ca₅(PO₄)₃(OH), usually written as Ca₁₀(PO₄)₆(OH)₂ to stress the fact that the lattice of the crystalline structure contains two molecules. Hydroxyapatite belongs to the crystallographic apatite family, which are isomorphic compounds having the same hexagonal structure. This compound has been used as a biomaterial for many years in various medical specialties.

Currently, hyaluronic acid or cross-linked versions thereof are used in various gel forms, for example as soft tissue augmentation products, adhesion barriers, and the like. Particularly, a PCT patent application WO2013055832 describes threads of cross-linked hyaluronic WO2011/109129 also discloses a method of manufacture of threads of crosslinked hyaluronic acid.

There is a need in the art to provide threads comprising hyaluronic acid in controlled manner and with improved desired properties, such as controlled degradation resistance, and/or spatial swelling behavior, and/or improved rheological properties and/or, mechanical stability, and/or reduced side-effects, and/or biocompatibility, and/or controlled osmolarity.

### Summary of the invention

In one aspect of the present invention there is provided a method of manufacturing a thread, comprising i) cross-linked hyaluronic acid, or one of its salts, at a concentration between 2% and 80% (W/W), and ii) hydroxyapatite at a concentration between 2% and 90% (W/W). The method comprises combining hyaluronic acid with a cross-linker and hydroxyapatite under cross-linking conditions as described herein to furnish precursor composition, forming a thread from said precursor composition, and drying the thread. The threads thus formed have an acceptable uniformity, e.g. low relative standard deviation of measurements of their diameter, swelling and/or mechanical properties.

In another aspect of the present invention there is provided a thread, comprising i) cross-linked hyaluronic acid, or one of its salts, and ii) hydroxyapatite. The threads have acceptable uniformity and mechanical properties, and exhibit acceptable swelling. The threads have lower water content, compared to their respective precursor compositions. The precursor compositions comprising the same components and water form another aspect of the invention.

It has been unexpectedly found by the present inventors that cross-linking of hyaluronic acid in presence of hydroxyapatite may be successfully performed. It has also been unexpectedly found that the addition of specific amount of hydroxyapatite to hyaluronic acid does not interfere with formation of acceptable threads, e.g. of high uniformity, acceptable mechanical strength and swelling characteristics. It has also been unexpectedly found that using particular cross-linking agents, e.g. ethylene glycol diglycidyl ether or poly(ethylene glycol) diglycidyl ether, it is possible to obtain significantly higher swelling of the threads without loss of mechanical properties. It has also been unexpectedly found that using hydroxyapatite particles of specific size, it is possible to control the swelling of the threads.

According to a method of manufacturing the thread of the invention, a precursor composition may be prepared, wherein hydroxyapatite may be added to hyaluronic acid before or during a cross-linking of the hyaluronic acid. It has now been unexpectedly found that the precursor composition thus prepared may furnish threads with uniform thickness, suitable for cosmetic and medical applications.

The diameter of the thread is essentially uniform. For example, the variation of the diameter expressed as relative standard deviation of diameter, e.g. of SEM measurements, taken along the length of the thread is less than 20%; e.g. representative measurements may be taken from at least 5 points, e.g. from at least 7 points, or at least 10 points. The points may be separated by about 0.5 mm apart. The uniformity of the threads may also be illustrated by the fact that measurable minimum diameter along the thread is at least 75 % of the measurable maximum diameter along said thread, excluding the edge effects. Preferably, the measurements may be taken from the central segment of the thread.

Further, the finished threads are relatively dry, i.e. may have between 0.5 and 33 weight percent of residual moisture.

The threads may be prepared from precursor composition comprising hyaluronic acid in concentrations from about 0.2 to 8 % w/w, inclusive. The precursor composition comprises calcium hydroxyapatite, e.g. between 0.5 and 20 %wt. In some embodiments, the concentration of calcium hydroxyapatite is between from about 0.5 and about 10 weight percent (%wt). In further embodiments, the concentration of calcium hydroxyapatite is between 8 to 20 %wt. The particles of hydroxyapatite may have an average particle size from 25 to 45 micrometers in size. Alternatively, hydroxyapatite particles may have above 50 micrometers, e.g. 45 to 100, in the average particle size.

The threads may be used in a cosmetic, medical (including surgical), or pharmaceutical application. The threads may further comprise additional material, e.g. drugs, non-limiting examples being local anesthetic, e.g. lidocaine, or hormones, growth factors, steroids.

In some embodiments, there is provided a process of manufacturing a thread of hyaluronic acid and calcium hydroxyapatite comprising: cross-linking in an aqueous medium hyaluronic acid or a salt thereof by adding a cross-linking agent and increasing the pH of the medium; neutralizing said aqueous medium; wherein calcium hydroxyapatite is present in said aqueous medium before said neutralizing, thereby providing a thread precursor composition; forming said precursor composition into a wet thread; and drying said wet thread to furnish a dry thread. Optionally, the process comprised adding a base to a reaction vessel which was previously charged with hyaluronic acid and a cross-linking agent, to form an alkaline reaction mixture, allowing the reaction mixture to commence cross-linking, adding and dispersing calcium hydroxyapatite into said reaction vessel, allowing said reaction mixture in said reaction vessel to complete cross-linking reaction, and neutralizing said aqueous medium by adding a buffering agent to bring the pH value to an essentially neutral value. In some embodiments, the cross-linking agent is selected from the group consisting of 1,4-butanediol diglycidyl ether, poly-(ethylene glycol) diglycidyl ether, and ethylene glycol diglycidyl ether. Preferably, the cross-linking agent is poly-(ethylene glycol) diglycidyl ether or ethylene glycol diglycidyl ether. The concentration of said hyaluronic acid in said aqueous medium may be between 0.2 and 8 weight percent. The concentration of calcium hydroxyapatite in said aqueous medium may be between 0.5 and 5 weight percent. The concentration of calcium hydroxyapatite in said aqueous medium may be between 5 and 20 weight percent. Said calcium hydroxyapatite may have a particle size distribution between 25 and 45 micrometers. The process comprises extruding said precursor composition through an orifice into a dehydration liquid or onto a drying surface. The process further comprises sterilizing the dried thread. The diameter of the thread is essentially uniform, the variation of the diameter expressed as relative standard deviation of diameter of SEM measurements taken along the length of the thread being less than 20%.

A thread precursor composition comprises cross-linked hyaluronic acid with calcium hydroxyapatite in an aqueous medium, wherein a concentration of calcium hydroxyapatite in said thread precursor composition may be between 0.5 and 20 weight percent. Preferably, the thread precursor composition may be manufactured according to any one of processes as described above.

A thread comprising cross-linked hyaluronic acid, hydroxyapatite, and less than 33 weight percent of water according to claim 1 is provided. The diameter of the thread is essentially uniform, the variation of the diameter expressed as relative standard deviation of diameter of SEM measurements taken along the length of the thread being less than 20%. A concentration of said hyaluronic acid in said thread may be between 15 and 50 weight percent. Said cross-linked hyaluronic acid may be cross-linked with a cross-linking agent, and said cross-linking agent is selected from the group consisting of 1,4-butanediol diglycidyl ether, poly-(ethylene glycol) diglycidyl ether, and ethylene glycol diglycidylether. The concentration of calcium hydroxyapatite in said thread may be between 15 and 25 weight percent. The concentration of calcium hydroxyapatite in said thread is between 45 and 65 weight percent. Said calcium hydroxyapatite may have a particle size distribution between 25 and 45 micrometers. Optionally, said thread is sterile.

### Brief descriptions of the drawings

Fig. 1a demonstrates a SEM image of a cross-linked hyaluronic acid thread.
Fig. 1b demonstrates a SEM image of a cross-linked hyaluronic acid thread with 23% of calcium hydroxyapatite.
Fig. 1c demonstrates a SEM image of a cross-linked hyaluronic acid thread with 36% of calcium hydroxyapatite.
Fig. 1d demonstrates a SEM image of a cross-linked hyaluronic acid thread with 54% of calcium hydroxyapatite.
Fig. 1e demonstrates a SEM image of a cross-linked hyaluronic acid thread with 64% of calcium hydroxyapatite.
Fig. 1f demonstrates a SEM image of a cross-linked hyaluronic acid thread with 68% of calcium hydroxyapatite.
Fig. 1g demonstrates a SEM image of a cross-linked hyaluronic acid thread with 86% of calcium hydroxyapatite.
Fig. 2a demonstrates a SEM image of a plurality of selected threads prepared according to the present invention.
Fig. 2b demonstrates a SEM image of a plurality of selected comparative threads.
Fig. 3a demonstrates a stress-strain curve of a plurality of threads prepared according to the present invention.
Fig. 3b demonstrates a stress-strain curve of a plurality of comparative threads.

### Detailed description of the invention

Generally, the threads of the present invention are manufactured by a process of cross-linking hyaluronic acid in an aqueous medium, in presence of calcium hydroxyapatite, i.e. calcium hydroxyapatite is added to the aqueous medium before the cross-linking reaction is substantially completed as described herein. Once the cross-linking is substantially completed, a precursor composition is formed, usually in form of a gel. The concentration of calcium hydroxyapatite in the precursor composition is therefore identical to the concentration in the aqueous medium of the cross-linking reaction, and the concentration of cross-linked hyaluronic acid is essentially identical to the combined concentrations of the free hyaluronic acid and the cross-linking agent. The precursor composition may then be formed in to a wet thread, e.g. by extrusion through a suitable orifice onto a substrate surface, optionally a shaped substrate, with desired surface properties, e.g. a concave or convex surface, e.g. onto stainless steel surface. The wet thread is then dried to form a dry thread.

In the context of the present invention, the terms "apatite", "hydroxyapatite", "calcium hydroxyapatite" and the like, as used herein, refer to a hydroxyapatite mineral of a general formula Ca₁₀(PO₄)₆(OH)₂, of a suitable quality and purity for use/administration, e.g. injection, in humans.

Although not part of the invention, hydroxyapatite may be substituted by other calcium phosphate minerals. The term "calcium phosphate minerals" refers to a family of minerals containing calcium ions (Ca²⁺) together with orthophosphates (PO₄³⁻), metaphosphates or pyrophosphates (P₂O₇⁴⁻) and occasionally hydrogen or hydroxide ions. Non-limiting examples for calcium phosphate minerals that may be used as an alternative to hydroxyapatite are alpha-tri-calcium phosphate and beta-tri-calcium phosphate. Other particles of biocompatible material may also be suitable.

Hydroxyapatite is embedded in the threads of the present invention as it may act as a dermal filling material, and may induce collagen synthesis. The inventors have now found that inclusion of hydroxyapatite into the threads may provide one or more of: an improved control of the degradability of the thread materials, their swelling behavior, their biocompatibility, their reduced side effects, and their controlled osmolarity.

Hydroxyapatite may be present in the threads according to the invention generally in concentrations between 2 and 90 %wt; in some embodiments the concentration is between 10 to 70 %wt. Hydroxyapatite may be provided in a form of a powder, e.g. a plurality of particles. The average particle size may be less than or equal to 650 µm, preferably less than about 200 µm, further preferably less than about 80 µm, and may also be less than about 500 nm. Further preferably about at least 75% of hydroxyapatite particles may be of a size between 25 µm and 500 µm, or between 25 µm and 300 µm, or between 25 µm and 200 µm, or between 25 µm and 100 µm, preferably between 25 µm and 45 µm. Alternatively or additionally, at least 75% of the hydroxyapatite particles may be between 1 µm and 100 µm, or between 5 µm and 45 µm, or between 10 µm and 45 µm. Alternatively or additionally, at least 75% of the hydroxyapatite particles may be between 30 µm and 400 µm, or between 35 µm and 255 µm, or between 45 µm and 100 µm. The terms "average particle size", "particle size", "weight average particle size" and the like, as used interchangeably herein in reference to the particles of calcium hydroxyapatite, refer to a weight average of a powder particle size distribution of calcium hydroxyapatite; i.e. the average value of particle size in a powder bulk taken by weight proportion of each fraction.

In the context of the present invention, the terms "hyaluronic acid", "HA" or "hyaluronate" refer interchangeably to a linear polysaccharide or to its salt, particularly to a nonsulfated glycosaminoglycan, composed of a repeated disaccharide units, each unit consisting of D-glucoronic acid and D-N-acetylglucosamine, via alternating β-1,4 and β-1,3 glycosidic bonds.

Hyaluronic acid or salts thereof may come from a variety of sources in a variety of molecular weights and other specifications. Generally, all sources of hyaluronic acid may be useful for the purposes of the present invention, including bacterial and avian sources.

The molecular weight of hyaluronic acid may be used as a characteristic to describe the material. The term "molecular weight" includes both the number-average molecular weight, and the weight-average molecular weight, as known for polymers. Useful hyaluronic acid materials may have a molecular weight of from about 0.25 MDa (mega Dalton) to about 4.0 MDa, e.g. from about 0.5 MDa to about 4.0 MDa. Useful ranges of the molecular weight of HA include from about 0.6 MDa to about 2.6 MDa, preferably from about 1.4 MDa to about 1.9 MDa. Useful ranges of the molecular weight of HA may also include from about 1.0 MDa to about 3.0 MDa, from about 1.0 MDa to about 2.5 MDa, from about 1.5 MDa to about 2.0 MDa. Specifically, HA of the molecular weight of about 0.7 MDa, of about 1.8 MDa, or of about 2.7 MDa may be used.

Hyaluronic acid may be further characterized with a polydispersity value, indicative of the variation of the molecular weights in the polymer. While it may be advantageous to use a low-polydispersity hyaluronic acid for the sake of improved repeatability of the processes, it may be economically infeasible. A reasonable compromise between the width of the molecular weights polydispersity and the price of the starting material may be achieved, and suitable hyaluronic acid materials may have a polydispersity from about 1.1 to 4.0, preferably less than 3.0, further preferably less than 2.0.

Hyaluronic acid may be at least partially cross-linked. The term "cross-linked" as used herein in reference to hyaluronic acid should be construed as chemical or physical modification of two or more polymer chains of hyaluronic acid, resulting in hyaluronic acid chains being bonded together, preferably covalently bonded. The process of cross-linking may preferably include a cross-linking agent. Similarly, a process of intermolecular or intramolecular reaction without a cross-linking agent, which results in a lactone, an anhydride, or an ester formation, either within a single polymer chain or between two or more chains, is herein referred to as "dehydration". The dehydration may occur concomitantly with cross-linking during the processes as described herein. The term "cross-linked" may also be used in reference to hyaluronic acid covalently linked to a cross-linking agent, or to a covalently modified hyaluronic acid.

The term "cross-linking agent" as used herein refers to molecules that contain at least two reactive functional groups that create covalent bonds between two or more molecules of hyaluronic acid. The cross-linking agents can be homo-bifunctional (i.e. have two reactive ends that are identical) or hetero-bifunctional (i.e. have two different reactive ends). The cross-linking agents suitable for use in the present invention usually comprise complementary functional groups to that of hyaluronic acid such that the cross-links could be formed. Preferably, the cross-linking does not form esterified hyaluronic acid. Non-limiting examples of cross-linking agents suitable for the present invention include 1,4-butanediol diglycidyl ether (BDDE), 1,2,7,8-diepoxyoctane (DEO), biscarbodiimide (BCDI), adipic dihydrazide (ADH), bis(sulfosuccinimidyl)suberate (BS3), hexamethylenediamine (NMDA), 1-(2,3-epoxypropyl)-2,3-epoxycyclohexane, multifunctional cross-linking agents such as pentaerythritol tetraglycidyl ether (PETGE) or PEG based such as polyethylene diglycidyl ether (PEGDE), mono ethylene glycol diglycidyl ether (EGDE), or a combination thereof. Preferably, the cross-linking agent is BDDE. Alternatively or additionally, the cross-linking agent is PEGDE or EGDE.

As used interchangeably herein, the terms "PEG-based cross-linking agent" and the like, refer to (poly)ethylene glycol derivatives. The term "PEG" refers to a polyethylene glycol polyether compound with many applications from industrial manufacturing to medicine. PEG is also known as polyethylene oxide (PEO) or polyoxyethylene (POE), depending on its molecular weight. The structure of PEG is commonly expressed as H-(O-CH₂-CH₂)ₙ-OH. Non-limiting examples of PEG derivatives that may be used as cross-linking agents are PEG epoxides, such as poly(ethylene glycol) diglycidyl ether, PEG-dihydrazide, PEG-dihalides, diazide-PEG, diaminooxy-PEG, diamine-PEG, etc.

The general term "cross-linking conditions" as used herein refers to reaction conditions that allow formation of covalent bonds between HA chains. Generally, cross-linking conditions include adjustment of the mixture to a desired pH and temperature, specific for a cross-linking agent used. The cross-linking conditions may include adjusting the pH of the mixture to a pH above 12 and exposing the mixture to 45°C for a first period, e.g. 1-5 hours, and then to 25 °C for a second period, e.g. 10-24 hours. The optimal cross-linking temperature and pH may be readily determined experimentally by testing the cross-linking conditions for HA that are well known in the art.

The weight concentration of the cross-linking agent in the aqueous medium may be between 0.05 %wt and 1 %wt.

Cross-linking of HA may be achieved by dissolving/dispersing hyaluronic acid in a solvent, preferably water, adding the cross-linking agent and hydroxyapatite, and bringing the mixture to cross-linking conditions. Alternatively, the cross-linking agent may be gradually added to a mixture of hyaluronic acid with optional additives, under cross-linking conditions.

The cross-linked HA with hydroxyapatite dispersed therein provides a precursor composition to the threads. The terms "thread" and the like, as used interchangeably herein, should be construed as an article of manufacture elongated along an imaginary length axis X, and having a diameter dimension essentially perpendicular to said length axis X. As used herein, the term "thread" refers to a long, thin, flexible form of a material. The thread as described herein can have a variety of shapes in the cross-section which are discussed below. The term "diameter" as used herein in reference to the threads, should be construed as the length value obtained along one of a plurality of imaginary thickness axes perpendicular to the imaginary length axis X of the thread, between the outmost boundaries of the thread along a thickness axis. Whereas the term "diameter" implies round circumference, in reference to the threads of the present invention the term should be construed as to include any length value along the thickness axis regardless the actual shape of the thread.

The diameter of the thread may usually have a relative standard deviation value of lower than 20%, e.g. between 0.5% and 10%, or between 0.5% and 15%, or between 0.5% and 20%, or between 0.5% and 9%, or between 1% and 8.5%, or between 1.5%, 2%, 2.5%, 3%, 3.5%, and 4%, or 9.0%, 8.5%, 8.0%, 7.5%, 7.0%, 6.5%, and 5.0%, or between 9.0%, 8.5%, 8.0%, 7.5%, 7.0%, 6.5%, and 6.0%. The diameter may be measured by any suitable technique, e.g. by scanning electron microscopy (SEM).

The thread may have a low amount of water, e.g. residual water. Generally, the thread may have less than 33 % of residual water, e.g. for a thread with relatively low amount of calcium hydroxyapatite, e.g. between 10 and 40 %, the thread may have between 10 and 33% of residual water, or for a thread with relatively high amount of calcium hydroxyapatite, e.g. between 45 and 75%wt, the thread may have between 10 and 20% of residual water. The terms "percent moisture", "residual water", "loss on drying" and the like, as used interchangeably herein, should be construed as the total percent of water by weight remaining in a thread. In one embodiment, the percent moisture of the thread is about 40 percent or less, or alternatively, about 30 percent or less, about 20 percent or less, or alternatively, about 10 percent or less. This can typically be measured by Karl Fisher titration. Alternatively, percent moisture can be also measured by weight loss of the threads upon drying to constant weight at 105 °C, i.e. 100% minus the ratio of the weight of a thread dried to constant weight to its weight before drying, or by measuring the amount of water that can be vacuum evaporated from the thread or by any other means to quantify the amount of water in the thread.

The thread may further comprise biologically active material, e.g. drugs. The non-limiting examples of drugs suitable for the composite materials include local anesthetic, e.g. lidocaine, and also hormones, growth factors, and steroids.

Preferably, the thread comprises:
i) residual water, between 10 and 33 %;
ii) cross-linked hyaluronic acid or one of its salts, at a concentration between 15 and 50 %wt (w/w);
iii) hydroxyapatite at a concentration between 10 and 75 %wt (w/w), as described herein; and optionally
iv) drug, for example a local anesthetic, such as lidocaine, up to 1 % w/w.

The thread may further comprise buffering agents and osmolarity agents, e.g. sodium chloride.

The precursor composition may usually comprise cross-linked hyaluronic acid or a salt thereof, and calcium hydroxyapatite. Preferably, the precursor composition comprises:
i) water;
ii) cross-linked hyaluronic acid or one of its salts, at a concentration between 0.2 and 8 %wt (w/w);
iii) hydroxyapatite at a concentration between 0.5 and 20 %wt (w/w), as described herein; and optionally
iv) drug, for example a local anesthetic, such as lidocaine, up to 1 % w/w.

The precursor composition may further comprise buffering agents and osmolarity agents, e.g. sodium chloride.

The concentration of hyaluronic acid in the precursor composition may range from 0.2 to 8 %wt, or from 1 to 6 %wt, or from 1.5 to 5 %wt, or from 2.5 to 4.5 wt%, or about 4 %wt. Preferably, hyaluronic acid concentration is between 1 and 4 % w/w.

The concentration of hydroxyapatite particles in the precursor composition may be between 0.5 and 5%wt, 0.7 and 4.5 %wt, or between 1 and 4 %wt, or between 1.5 and 3.5 %wt, between 1.7 and 3 %wt, or between 2 and 3 %wt, or between 2.2 and 2.7 %wt. According to one embodiment, calcium hydroxyapatite particles are present in the precursor composition at a concentration of 2.5% w/w.

Alternatively, the concentration of hydroxyapatite particles in the precursor composition may be between 5 and 15%wt, 6 and 14 %wt, or between 6.5 and 13 %wt, or between 7 and 12 %wt, between 7.5 and 12 %wt, or between 8.5 and 11.5 %wt, or between 9 and 11 %wt. According to one embodiment, calcium hydroxyapatite particles are present in the precursor composition at a concentration of 10% w/w.

The concentration of hydroxyapatite particles in the thread may be between 2 and 90 %wt, e.g. 10 and 75 %wt. In some embodiments the concentration may be between 10 to 30 %wt, or, between 15 and 25%wt, or between 17 and 23% %wt, or between 18.5 and 21.5 %wt. In further embodiments, the concentration may be between 40 and 75 %wt, or between 45 and 70 %wt, or between 47.5 and 65 %wt, or between 50 and 62.5 %wt, or between 55 and 62.5 %wt, or between 55 and 60 %wt.

The thread of the present invention may be used in a pharmaceutical, a medical or a cosmetic application.

The precursor composition may be prepared by a method comprising the steps of: a) preparation of a mixture comprising water and at least 0.2% to 8% w/w of hyaluronic acid or a salt thereof, and a cross-linking agent; b) addition of hydroxyapatite at a concentration between 0.5%wt to 15%wt, and homogeneously dispersing it in the hyaluronic acid, and c) exposing the mixture to cross-linking conditions to obtain a precursor gel.

The precursor compositions may be prepared by a method comprising the steps of: a) preparation of a first mixture comprising water and at least 0.2% to 8% weight of hyaluronic acid or a salt thereof, and a cross-linking agent; b) exposing the mixture to cross-linking conditions; c) addition of hydroxyapatite at a concentration between 0.5 %wt to 20 %wt, d) further exposing the mixture to cross-linking conditions, and e) neutralizing the mixture.

The pH and osmolarity of the precursor composition may further be adjusted to physiological values. Neutralization may be carried out by addition of aqueous solutions comprising pharmaceutically acceptable buffering agents, e.g. phosphate salts, of pH between 6 and 8, according to the requirement of the final pH.

Similarly, osmolarity adjustment may be performed by adding to the mixture a solution of salts, e.g. sodium chloride, and mixing the formulation to obtain homogeneous gel.

At any step of the manufacturing process, the mixture may be tested for quality assurance purposes. The applicable standard tests are known to a technically skilled person and include e.g. rheometry, pH determination, residual cross-linking agent quantification, microscopy, sedimentation rate by centrifugation, and others.

The ready precursor composition may be milled to, e.g. by extrusion, or by a high-shear mixer, to improve the flow properties prior to forming threads. The milling may be performed in presence of additional liquid constituents, e.g. water and/or neutralization and/or osmolarity adjustment solution.

The precursor composition may be fed into syringes and sterilized, e.g. by autoclaving, or by gamma irradiation, prior to forming of threads. Alternatively, the threads may be formed and dried, and then sterilized.

The sterile threads may be used in a variety of applications, e.g. in tissue filling, such as wrinkle filling, suturing, or bone graft filling, stretch marks (striae) attenuation, scars attenuation, face lifts, tissue remodeling and augmentation, drug delivery, mesotherapy, biorevitalizing, and surgery.

In some embodiments, the threads are formed by extruding a precursor composition through an orifice with the desired shape and dimensions. The formed threads may be further shaped according to the need, and finally dried. Thus, any method that enables extrusion of the precursor composition through an orifice with the desired shape and dimensions can be used to form the threads of the present invention.

In some embodiments, the threads may be formed by forming the precursor composition into a film and drying the formed composition. Shaping the formed film into threads may be performed by cutting threads from the film.

The threads as described herein can be made into a variety of shapes. The term "substantially cylindrical" refers to a thread wherein the cross-section of the thread is round. The term "substantially" as used herein refers to shapes of the threads that at least 50 percent of the thread has the approximate shape described. The term substantially is also used to encompass threads which have a variety of shapes along the length of the thread. For example, a thread could be substantially cylindrical but the ends of the thread may be tapered. In some embodiments, the substantially cylindrical threads can be provided when the contact angle of the gel composition and the substrate on which it is extruded have an equilibrium contact angle of greater than about 90 degrees.

The term "substantially D-shaped" refers to a thread wherein the cross-section is lenticular, e.g. D-shaped or substantially semi-circular. The substantially D-shaped threads have one flat side and one substantially round side. In some embodiments, the substantially D-shaped threads can be provided when the contact angle of the gel composition and the substrate on which it is extruded have an equilibrium contact angle of about 90 degrees.

The term "substantially ribbon-shaped" refers to a thread wherein the thickness of the thread is less than about 50 percent of the width of the thread. In some embodiments, the cross-section is substantially rectangular or oval. In some embodiments, the ribbon-shaped threads can be provided when the contact angle of the gel composition and the substrate on which it is extruded have an equilibrium contact angle of less than about 90 degrees. Alternatively, the ribbon-shaped threads can be formed by cutting a wet gel to achieve the desired cross-sectional shape. "Ribbon-shaped" may also include shapes that are substantially ellipsoidal. The term "substantially ellipsoidal" refers to a thread wherein the cross-section is substantially oblong or elliptical.

In one aspect, the threads may be formed by providing the precursor composition in a closed compartment having an outlet and by applying pressure to the closed compartment while the outlet remains open thus extruding the precursor composition in the form of a thread.

In one aspect, there is provided a thread comprising hyaluronic acid and hydroxyapatite, wherein at least a portion of the hyaluronic acid is substantially cross-linked.

In one embodiment, the thread comprises HA in a concentration of 10% and 60% (W/W), and hydroxyapatite at a concentration between 10% and 75% (W/W).

In another embodiment, the thread comprises HA in a concentration of between 2% and 80% (W/W) and between 2% and 90% (W/W) CaHAp.

In another embodiment, the thread comprises HA in a concentration of between 5% and 40% (W/W) and between 10% and 80% (W/W) CaHAp.

In another embodiment, the thread comprises HA in a concentration of between 10% and 30% (W/W) and between 30% and 60% (W/W) CaHAp.

In another embodiment, the thread comprises HA in a concentration of between 15% and 25% (W/W) and between 45% and 55% (W/W) CaHAp.

In another embodiment, the thread comprises HA in a concentration of between 2% and 50% (W/W) and between 10% and 80% (W/W) CaHAp.

In one embodiment, the thread has a diameter of at least about 0.01 mm. In one embodiment, the thread has a diameter of between 0.05 to 0.2 mm, 0.4 to 0.6 mm, between 0.35 to 0.65 mm, between 0.3 to 0.7 mm, between 0.2 to 0.8 mm, between 0.1 to 0.6 mm, between 0.1 to 0.8 mm, between 0.1 to 1 mm, between 0.1 to 2 mm, between 0.1 to 3 mm, between 0.3 to 1 mm, between 0.3 to 2 mm or between 0.3 to 3 mm.

The term "swelling capacity" as used herein refers to the capacity of the dry thread to absorb water (or an aqueous solution). In some embodiments, the dry thread can absorb water (or an aqueous solution) to about 50 times its weight, 30 times its weight, 10 times its weight, 5 times its weight, 2 times its weight. In yet another embodiment, the thread does not absorb water at all and thus does not swell.

In another embodiment, the thread elongates in the tissue up to 400% its length, up to 300% its length, up to 200% its length, up to 150% its length, up to 120% its length, or none at all.

In yet another aspect, there is provided a thread comprising cross-linked hyaluronic acid and hydroxyapatite prepared by the steps of: a) preparing a precursor composition comprising cross-linked HA and calcium hydroxyapatite; b) extruding the precursor composition to form a wet thread; and c) drying the wet thread to form a dry thread.

In another aspect, there is provided a thread comprising cross-linked hyaluronic acid and hydroxyapatite, prepared by the steps of: a) preparing a precursor composition comprising cross-linked HA and CaHAp formulation b) extruding the precursor composition to form a wet thread; c) drying the wet thread to form a dry thread; d) contacting the dried thread with a neutralizing solution; and e) re-drying the neutralized thread.

In one embodiment, the drying may be carried out in controlled conditions, e.g. 25°C and 50% relative humidity. The drying time may usually be less than 10 minutes, less than 30 minutes, less than 1 hour, less than 2 hours, less than 3 hours, less than 4 hours, less than 6 hours, less than 10 hours, less than 12 hours, less than 24 hours, less than 48 hours or more than 48 hours, or between 5 and 10 minutes, between 5 and 30 minutes, between 10 and 30 minutes, between 10 and 60 minutes, between 30 and 60 minutes, between 30 and 120 minutes, and between 60 and 120 minutes.

In one embodiment, there is provided a thread according to any of the above embodiments, wherein the thread is terminally sterilized.

Some specific embodiments of the present invention may be demonstrated by the following examples, which are not intended to limit it in any respect.

### EXAMPLES

Generally, the threads were prepared in two basic steps: i) preparation of a HA and CaHAp precursor composition, and ii) preparation of threads from the precursor composition.

### Example 1 - Hyaluronic acid and hydroxyapatite threads

Sodium hyaluronate (HA) of molecular weight 1.3-2.0 MDa (labeled 1.3 MDa, pharma grade, 7.1% water, supplied by Bloomage Freda), 1.60 g, was added to 13.55 g water, followed by 0.157 g of 1,4-butanediol diglycidyl ether (BDDE) (supplied by TCI), thoroughly mixed with a spatula and then to dissolution at room temperature, at 2000 rpm using a Thinky planetary mixer model ARM-310 for 2 minutes. The solution was then allowed to incubate at 25°C for 30 minutes.

Thereafter, 2696 mg of 1M sodium hydroxide (NaOH)solution were added, was added to the mixture, bringing the pH >12. The mixture was then homogenized for 10 min at 2000 rpm using the centrifugal mixer. The solution was then allowed to incubate at 25°C for further 120 minutes.

Calcium hydroxyapatite Ca₁₀(PO₄)₆(OH)₂ dense microspheres (medical grade), with an average particle size of 25-45 micrometers, 4.00 g, was added to the mixture, and then mixed again for 10 min at 2000 rpm. The mixture was then placed in an oven set to 45 °C for 3 hours, and then placed at 25 °C for additional 18 hours.

The mixture was neutralized by forcing the cross-linked gel through a 50-mL syringe into neutralization solution. The composition of the neutralization solution was 0.084 g of potassium dihydrogen phosphate (KH₂PO₄) (Pharma grade, supplied by Merck), 0.295 g of disodium hydrogen phosphate (Na₂HPO₄) (Pharma grade, supplied by Merck), 15.71 g of water for injection, and 1.92 g of 1M hydrochloric acid (HCl) solution (Pharma grade, supplied by Merck). The mixture was then mixed for 10 minutes at 2000 rpm using the centrifugal mixer. The gel was finally degassed *in vacuo,* by subjecting it to 20 mbar vacuum for 30 minutes, and transferred into 50-mL syringe.

The bulk was then extruded through 16G or 19G blunt needles onto a polypropylene surface to create threads by moving the syringe relative to the surface while pushing the plunger, at about 1 mm/second. The threads were dried for about 20 hours at 25 °C and 50% relative humidity in climate-controlled incubator (Binder KBF-720).

The precursor composition contained about 3.7 % of cross-linked hyaluronic acid, about 10 % of calcium hydroxyapatite, and about 84.5% of water, and the threads contained about 20.9% of cross-linked hyaluronic acid, about 56.3% of calcium hydroxyapatite, and about 12.8% of residual water.

The threads were then wetted with deionized water for more than 20 minutes before measuring their weights and lengths. These measurements are summarized in the Table 1 below.

**Table 1**

| | | Immediately after extrusion | | After drying | | After wetting | |
|---|---|---|---|---|---|---|---|
| Needle | Thread no. | Length (mm) | Weight (mg) | Length (mm) | Weight (mg) | Length (mm) | Weight (mg) |
| 16G | 1 | 118 | 360 | 109 | 63.2 | 176 | 286 |
| | 2 | 117 | 324.1 | 102 | 58.7 | 166 | 259 |
| | 3 | 111 | 303.0 | 96 | 54.0 | 149 | 225 |
| 19G | 1 | 108 | 84.5 | 102 | 15.4 | 167 | 65.7 |
| | 2 | 127 | 116 | 116 | 20.5 | 187 | 101.8 |
| | 3 | 112 | 101 | 103 | 17.3 | 166 | 85.2 |

### Example 2 - varying amount of hydroxyapatite in the threads

Threads were prepared from precursors comprising 0 to 30% of hydroxyapatite according to the following procedure: BDDE was added to water, followed by HA powder. The mixture was manually mixed, followed by 2 minutes mixing using Thinky mixer (at 2000 rpm). After 30 minutes, 1 M NaOH solution was added and mixed, first manually, and then 2 minutes by Thinky mixer (at 2000 RPM). Both mixing steps were repeated. The mixture was then mixed for 1 hour by with a Rotogen mixing device (Collomix). The mixture was mixed manually with the suitable amounts of CaHAp, followed by Thinky (4 min 2000 rpm). The mixtures were then placed in 45°C for 3 hours, followed by 15 hours in 25°C. The mixtures were then neutralized with the neutralization solution and degassed. Threads were obtained by extruding the precursor through a 19G nozzle onto stainless steel surface, followed by 2 hours drying in 25°C and 50% relative humidity.

The amounts of the ingredients were as described in the example 1, with varying amount of calcium. The thread having no calcium hydroxyapatite is not part of this invention. The characteristics of the threads thus obtained are given as averages ± standard deviation, in the Table 2. The amounts of the ingredients are summarized in the table 3.

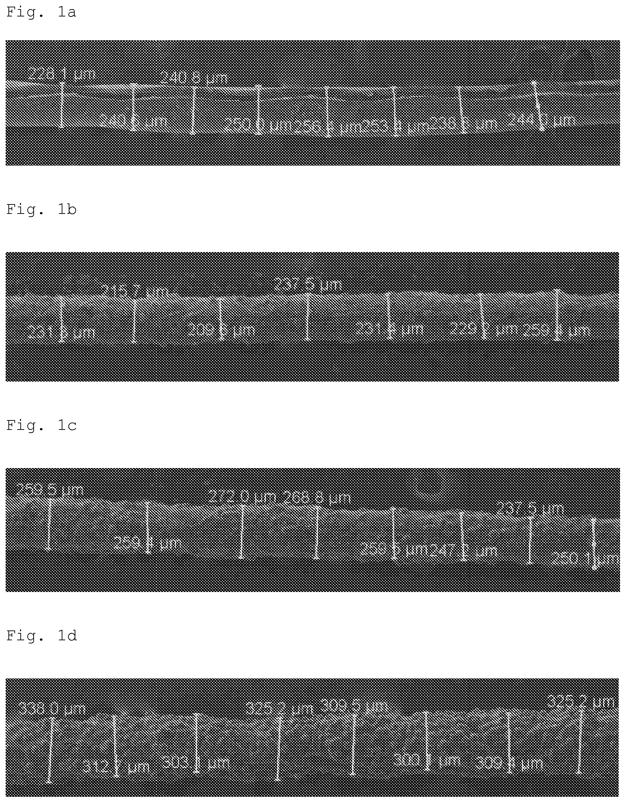

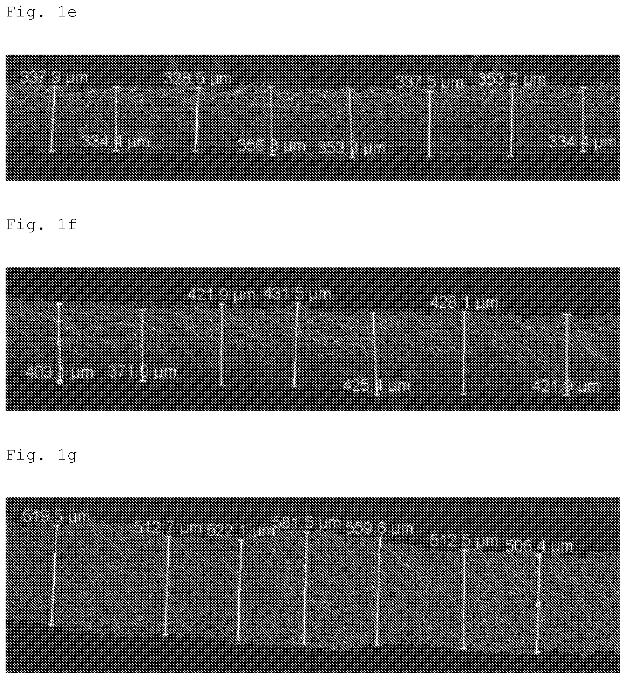

The scanning electron micrographs of the threads are shown in the Figures 1a-1f.

**Table 2**

| CaHAp % | % Elongation | % Swelling | Tensile strength (N) | Diameter | % RSD of diameter |
|---|---|---|---|---|---|
| 0% | 30±0.88 | 321±11.05 | 1.43±0.22 | 236±17 | 7.2 |
| 2.5% | 33±0.54 | 222±12.46 | 1.22±0.08 | 255±23 | 9.1 |
| 5% | 33±0.97 | 262±13.04 | 1.22±0.08 | 267±16 | 6.0 |
| 10% | 33±1.60 | 154±10.97 | 0.87±0.05 | 315±28 | 8.9 |
| 15% | 36±0.73 | 151±2.87 | 0.82±0.08 | 339±32 | 9.4 |
| 20% | 36+2.67 | 135±9.02 | 0.70±0.07 | 417±34 | 8.2 |
| 30% | 36±0.62 | 125±6.55 | 0.56±0.15 | 527±25 | 4.7 |

**Table 3**

| % drying | Calcium hydroxyapatite | | Water | | Hyaluronic acid | |
|---|---|---|---|---|---|---|
| | Precursor | Thread | Precursor | Thread | Precursor | Thread |
| 91.0±0.2 | 0% | 0 | 94.21 | 35.7 | 4.00 | 44.4 |
| 89.1±0.3 | 2.5 % | 22.9 | 91.83 | | 3.99 | 36.6 |
| 85.9±0.4 | 5% | 35.5 | 89.50 | 25.5 | 3.8 | 27.0 |
| 81.5±0.5 | 10% | 54.1 | 84.79 | 17.8 | 3.6 | 19.5 |
| 76.6±0.3 | 15% | 64.1 | 80.08 | 14.9 | 3.4 | 14.5 |
| 70.4±0.5 | 20% | 67.6 | 75.37 | 16.8 | 3.2 | 10.8 |
| 65.0±0.3 | 30% | 85.7 | 65.95 | 2.7 | 2.8 | 8.0 |

Tensile strength was measured using a tensile strength measurement instrument (Dillon model GL050) attached to a universal testing machine (IMADA MX2-110-s), by stretching single 100-mm long dry thread at speed of 45 mm/sec. Thread diameter was measured on five specimens per thread using scanning electron microscopy. Seven measurements were performed on each specimen, at points spaced about 0.5 mm apart along a section of about 3.5 mm of the thread specimen.

It can be seen that although the presence of hydroxyapatite reduces the strength and the swelling capacity of the threads, there is unexpectedly a concentration range of hydroxyapatite wherein both values are still acceptable for intended applications.

It can be equally readily seen that the relative standard deviation of the thickness (i.e. diameter) of the threads is very low, indicating homogeneity of the thread.

### Examples 3A (of the invention), 3B (comparative based on US patent application 2015/0257989), and 3C (comparative based on PCT patent application WO2013055832) - the effect of the process

Thread precursor compositions were prepared according to three different procedures. The precursor compositions contained 3.6 % of hyaluronic acid and 10% of calcium hydroxyapatite.

Threads 3.A were prepared according to Example 2. Briefly, in the threads of comparative example 3.B calcium hydroxyapatite was added after the neutralization step; the comparative example 3.C is devoid of calcium hydroxyapatite.

The precursors of threads 3.B were prepared according to the procedure in the Example 1 of US patent application 2015/0257989, to Anteis S.A and dried as described in example 2. In brief, HA was mixed with NaOH (Rotogen, 300 RPM, 90 minutes), then, BDDE was added and mixed gently and the mixture was placed in 50°C for 2 hours. HCl 1M solution was then added and the mixture was mixed using an overhead stirrer (5 minutes, 70 RPM). The mixture was then dialyzed in a neutralization solution for 24 hours. Additional neutralization solution was added to reach final HA concentration, and was mixed (Rotogen, 300 RPM, 90 minutes). CaHAp was then added, 10 %wt, mixed (Rotogen, 300 RPM, 90 minutes) and degassed.

The precursors of threads 3.C were prepared according to the adapted procedure in the Example 1 of PCT patent application WO2013055832, to Tautona Group LP and dried as described in example 2. In brief, HA powder is hydrated in about 75% of a total volume of NaOH for about 30 minutes at about 50°C. The hydrated HA was then added to a syringe and mixed thoroughly (syringe-to-syringe about 20 times). Heating was continued for approximately 30 minutes. The cross-linker (BDDE) was then dissolved in the remaining portion of the total volume of NaOH (25% of the total volume), added to the hydrated hyaluronic acid solution, mixed thoroughly (syringe-to-syringe about 20 times), heated for about 30 minutes and re-mixed (syringe-to-syringe about 20 times). Heating was then continued at about 50° C for an additional 3 to 5 hours.

Threads were dried as described in the Example 2. Thread precursor composition 3.C failed to furnish threads, as it was too soft and could not be processed into threads.

Threads from precursor compositions 3.A and 3.B were evaluated as in the Example 2.

Ten specimens of each type were evaluated in swelling testing, and five specimens for mechanical testing. Diameter was measured as in the Example 2 at points spaced about 0.5 mm apart, along about 7 mm sections.

The results are summarized in the table 4 below. The values are given as averages ± standard deviation.

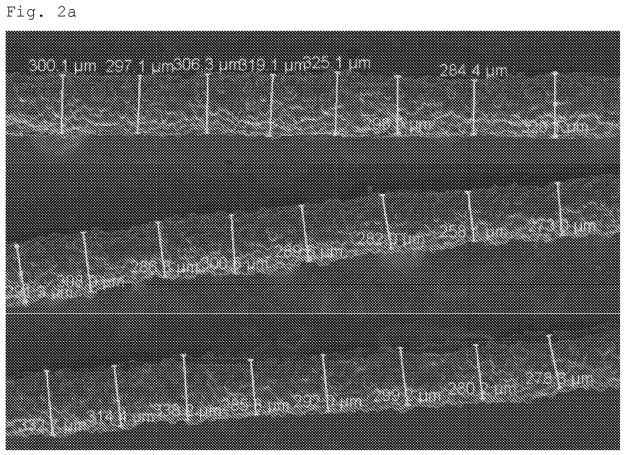

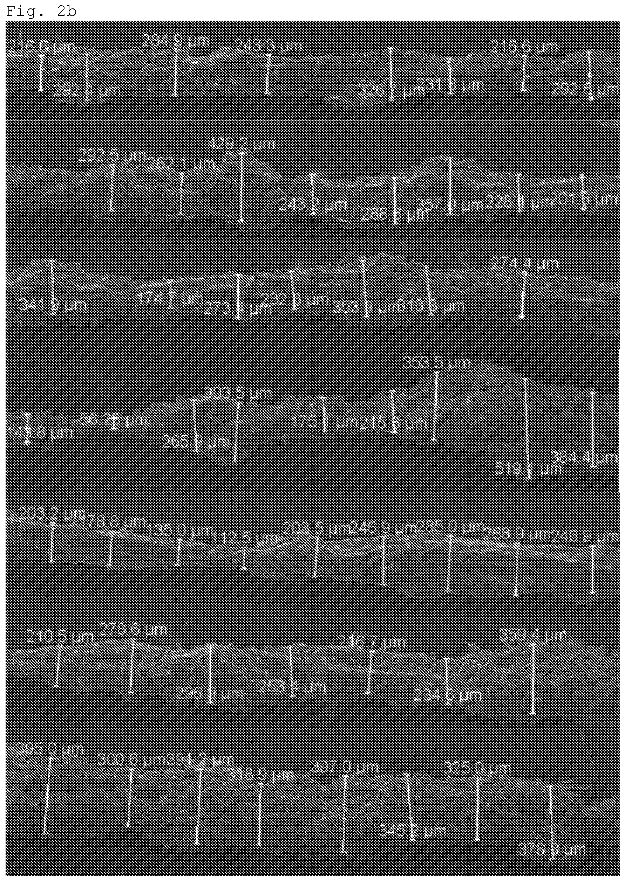

The scanning electron micrographs of selected threads are shown in the Figures 2a and 2b. Figure 2a shows 3.A threads of the present invention, and figure 2b shows 3.B threads of the comparative art.

**Table 4**

| Thread | % Elongation | % Swelling | Tensile strength (N) | diameter | % RSD diameter |
|---|---|---|---|---|---|
| 3.A | 32±0.42 | 145 ±3.25 | 0.87±0.05 | 295±19 | 6.4 |
| 3.B | 8.42±0.41 | 62.4±11.35 | 0.49±0.27 | 272±81 | 29.8 |
| 3.C | No thread was obtained | | | | |

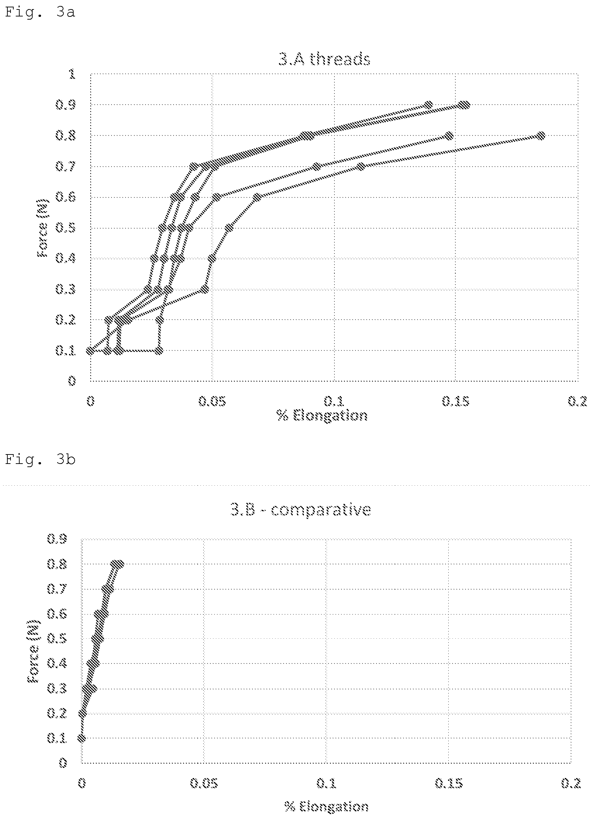

The stress-strain curves of the threads are presented in the Figure 3. It can be seen that threads exhibited different behavior upon stretching. As 3.A threads exhibited plastic elongation before failure, the 3.B threads were more brittle. Moreover, the uniformity in 3.A threads has resulted in lower RSD in the maximum tensile strength value, as opposed to the highly variable 3.B threads.

As can be observed from the SEM images and from the high standard deviation value in the threads' average diameter, the 3.B threads are less uniform in term of their diameter and the hydroxyapatite particles' distribution in the thread. The lack of uniformity in the 3.B thread, reflected in the high RSD values, leads to it having more weak points and propensity to fail under reasonable use.

### Example 4 - the effect of particle size of hydroxyapatite on the threads

The threads with different hydroxyapatite size were prepared and examined. The threads were prepared according to the procedure and the composition in the Example 2, with 10% of calcium hydroxyapatite. The particles had a weight average size as follows: 4.a) ∼12 µm spheres; 4.b) 25-45 µm spheres, as in the Example 2, and 4.c) 45-100 µm spheres.

The results are summarized in the table 5 below. The values are given as averages ± standard deviation. Five specimens were used. Diameter was calculated from about 3.5 mm long segments every 0.5 mm.

**Table 5**

| Thread | % Elongation | % Swelling | Tensile strength (N) | Diameter | % RSD diameter |
|---|---|---|---|---|---|
| 4.A | 26±1.60 | 147±12.31 | 1.16±0.15 | 278±20 | 7.2 |
| 4.B | 32±0.42 | 145±3.25 | 0.87±0.05 | 295±19 | 6.4 |
| 4.C | 42±1.29 | 449±11.61 | 0.62±0.08 | 293±31 | 10.6 |

It can be readily seen that hydroxyapatite particles affect the thread's characteristics. The larger the particles are, the weaker the thread, and it also elongates more. It can be equally readily seen that threads comprising larger particles exhibited unexpectedly high swelling capacity. It can be seen that particles with the weight average size between 25 and 45 micrometers provide the most homogeneous threads.

### Example 5 - Preparation of threads with different cross-linkers

The threads were prepared according to the formulation and procedure of the Example 2. Precursor composition 5.A contained 1,4-Butanediol diglycidyl ether BDDE for cross-linker; 5.B Poly-(ethylene glycol) diglycidyl ether (PEG-DE, supplied by Sigma); and 5.C Ethylene Glycol Diglycidyl Ether (EG-DE, supplied by TCI) .

The results are summarized in the table 6 below. The values are given as averages ± standard deviation. Five specimens were used. Diameter was calculated from about 7.0 mm long segments every 0.5 mm.

**Table 6**

| Thread | % Elongation | % Swelling | Diameter | % RSD diameter |
|---|---|---|---|---|
| 5.A | 32±0.42 | 145±3.25 | 295±19 | 6.4 |
| 5.B | 51±3.56 | 278±14.50 | 329±24 | 7.3 |
| 5.C | 55±3.14 | 303±22.01 | 421±41 | 9.7 |

It can be readily seen that all threads were homogeneous. Nevertheless, even though all threads were manufactured in the same manner using the same formulation and process, the threads comprising ethylene glycol linkers unexpectedly exhibited significantly increased elongation and swelling capacity without loss of the mechanical strength of the thread.

### Example 6- Preparation of threads by hanging and air drying

The precursor composition obtained as in Example 1 was placed in a 1-mL syringe with a nozzle of a 21G needle. The syringe was secured with the nozzle facing slightly downwards, to allow the gravity to pull down expelled precursor composition. About 50 mg of the precursor composition were injected onto a flat surface, and a thread was formed a thread by elevating the syringe to 10 cm height. The thread was allowed to dry at air. Resulting thread was thin, round, and uniform.

## Claims

1. A thread comprising cross-linked hyaluronic acid, calcium hydroxyapatite, and less than 33 weight percent of water, wherein the diameter of the thread is essentially uniform, the variation of the diameter expressed as relative standard deviation of diameter of SEM measurements taken along the length of the thread being less than 20%.

2. The thread of claim 1, wherein a concentration of said hyaluronic acid in said thread is between 15 and 50 weight percent.

3. The thread of any one claims 1 or 2, wherein said cross-linked hyaluronic acid is cross-linked with a cross-linking agent, and said cross-linking agent is selected from the group consisting of 1,4-butanediol diglycidyl ether, poly-(ethylene glycol) diglycidyl ether, and ethylene glycol diglycidylether.

4. The thread of any one of claims 1 to 3, wherein a concentration of calcium hydroxyapatite in said thread is between 10 and 35 weight percent.

5. The thread of any one of claims 1 to 3, wherein a concentration of calcium hydroxyapatite in said thread is between 35 and 75 weight percent.
[001]

6. The thread of any one of preceding claims, wherein said calcium hydroxyapatite has a weight average particle size distribution between 25 and 45 micrometers.

7. A process of manufacturing a thread as defined in claim 1, the process comprising:
cross-linking in an aqueous medium hyaluronic acid or a salt thereof by adding a cross-linking agent and increasing the pH of the medium;
neutralizing said aqueous medium;
wherein hydroxyapatite is added to hyaluronic acid before or during cross-linking of the hyaluronic acid,
thereby providing a thread precursor composition;
forming said precursor composition into a wet thread; and
drying said wet thread to furnish a dry thread.

8. The process of claim 7, comprising adding a base to a reaction vessel which was previously charged with hyaluronic acid and a cross-linking agent, to form an alkaline reaction mixture, allowing the reaction mixture to commence cross-linking, adding and dispersing calcium hydroxyapatite into said reaction vessel, allowing said reaction mixture in said reaction vessel to complete cross-linking reaction, and neutralizing said aqueous medium by adding a buffering agent to bring the pH value to an essentially neutral value.

9. The process of any one claims 7 or 8, wherein said cross-linking agent is selected from the group consisting of 1,4-butanediol diglycidyl ether, poly-(ethylene glycol) diglycidyl ether, and ethylene glycol diglycidyl ether.

10. The process of any one of claims 7 to 9, wherein a concentration of said hyaluronic acid in said aqueous medium is between 0.2 and 8 weight percent.

11. The process of any one of claims 7 to 10, wherein a concentration of calcium hydroxyapatite in said aqueous medium is between 0.5 and 5 weight percent.

12. The process of any one of claims 7 to 10, wherein a concentration of calcium hydroxyapatite in said aqueous medium is between 5 and 20 weight percent.

13. The process of any one of claims 7 to 12, wherein said calcium hydroxyapatite has a weight average particle size distribution between 25 and 45 micrometers.

14. The process of any one of claims 7 to 13, wherein said forming comprises extruding said precursor composition through an orifice into a dehydration liquid or onto a drying surface.

15. The process of any one claims 7 to 14, wherein the diameter of the thread is essentially uniform, the variation of the diameter expressed as relative standard deviation of diameter of SEM measurements taken along the length of the thread being less than 20%.

## Patentansprüche

1. Faden, umfassend vernetzte Hyaluronsäure, Calcium-Hydroxyapatit und weniger als 33 Gewichtsprozent Wasser, wobei der Durchmesser des Fadens im Wesentlichen gleichmäßig ist, wobei die Abweichung des Durchmessers, ausgedrückt als relative Standardabweichung des Durchmessers von REM-Messungen, die entlang der Länge des Fadens vorgenommen wurden, weniger als 20 % beträgt.

2. Faden nach Anspruch 1, wobei eine Konzentration der Hyaluronsäure in dem Faden zwischen 15 und 50 Gewichtsprozent liegt.

3. Faden nach einem der Ansprüche 1 oder 2, wobei die vernetzte Hyaluronsäure mit einem Vernetzungsmittel vernetzt ist und das Vernetzungsmittel aus der Gruppe ausgewählt ist, die aus 1,4-Butandioldiglycidylether, Poly-(ethylenglycol)diglycidylether und Ethylenglycoldiglycidylether besteht.

4. Faden nach einem der Ansprüche 1 bis 3, wobei eine Konzentration von Calcium-Hydroxyapatit in dem Faden zwischen 10 und 35 Gewichtsprozent liegt.

5. Faden nach einem der Ansprüche 1 bis 3, wobei eine Konzentration von Calcium-Hydroxyapatit in dem Faden zwischen 35 und 75 Gewichtsprozent liegt.

6. Faden nach einem der vorstehenden Ansprüche, wobei das Calcium-Hydroxyapatit eine gewichtsmittlere Partikelgrößenverteilung zwischen 25 und 45 Mikrometer aufweist.

7. Verfahren zum Herstellen eines Fadens nach Anspruch 1, wobei das Verfahren Folgendes umfasst:
Vernetzen von Hyaluronsäure oder einem Salz davon in einem wässrigen Medium durch Hinzufügen eines Vernetzungsmittels und Erhöhen des pH-Werts des Mediums;
Neutralisieren des wässrigen Mediums;
wobei Hydroxyapatit zur Hyaluronsäure vor oder während des Vernetzens der Hyaluronsäure hinzugegeben wird,
wodurch eine Faden-Vorläuferzusammensetzung bereitgestellt wird;
Formen der Vorläuferzusammensetzung zu einem nassen Faden; und
Trocknen des nassen Fadens, um einen trockenen Faden bereitzustellen.

8. Verfahren nach Anspruch 7, umfassend das Hinzufügen einer Base zu einem Reaktionsgefäß, das zuvor mit Hyaluronsäure und einem Vernetzungsmittel beladen wurde, um ein alkalisches Reaktionsgemisch zu bilden, Zulassen, dass das Reaktionsgemisch sich zu vernetzen beginnt, Hinzufügen von Calcium-Hydroxyapatit in das Reaktionsgefäß und Verteilen, Zulassen, dass das Reaktionsgemisch in dem Reaktionsgefäß die Vernetzungsreaktion abschließt, und Neutralisieren des wässrigen Mediums durch Zugeben einer Puffersubstanz, um den pH-Wert auf einen im Wesentlichen neutralen Wert zu bringen.

9. Verfahren nach einem der Ansprüche 7 oder 8, wobei das Vernetzungsmittel aus der Gruppe ausgewählt ist, die aus 1,4-Butandioldiglycidylether, Poly-(ethylenglycol)diglycidylether und Ethylenglycoldiglycidylether besteht.

10. Verfahren nach einem der Ansprüche 7 bis 9, wobei eine Konzentration der Hyaluronsäure in dem wässrigen Medium zwischen 0,2 und 8 Gewichtsprozent liegt.

11. Verfahren nach einem der Ansprüche 7 bis 10, wobei eine Konzentration von Calcium-Hydroxyapatit in dem wässrigen Medium zwischen 0,5 und 5 Gewichtsprozent liegt.

12. Verfahren nach einem der Ansprüche 7 bis 10, wobei eine Konzentration von Calcium-Hydroxyapatit in dem wässrigen Medium zwischen 5 und 20 Gewichtsprozent liegt.

13. Verfahren nach einem der Ansprüche 7 bis 12, wobei das Calcium-Hydroxyapatit eine gewichtsmittlere Partikelgrößenverteilung zwischen 25 und 45 Mikrometer aufweist.

14. Verfahren nach einem der Ansprüche 7 bis 13, wobei das Formen das Extrudieren der Vorläuferzusammensetzung durch eine Öffnung in eine Entwässerungsflüssigkeit oder auf eine Trockenfläche umfasst.

15. Verfahren nach einem der Ansprüche 7 bis 14, wobei der Durchmesser des Fadens im Wesentlichen gleichmäßig ist, wobei die Abweichung des Durchmessers, ausgedrückt als relative Standardabweichung des Durchmessers von REM-Messungen, die entlang der Länge des Fadens vorgenommen wurden, weniger als 20 % beträgt.

## Revendications

1. Fil comprenant un acide hyaluronique réticulé, de l'hydroxyapatite de calcium, et moins de 33 % massique d'eau, dans lequel le diamètre du fil est essentiellement uniforme, la variation du diamètre exprimée en tant qu'écart-type relatif du diamètre de mesures SEM prises le long de la longueur du fil étant inférieure à 20 %.

2. Fil selon la revendication 1, dans lequel une concentration dudit acide hyaluronique dans ledit fil se situe entre 15 et 50 % massique.

3. Fil selon l'une quelconque des revendications 1 ou 2, dans lequel ledit acide hyaluronique réticulé est réticulé avec un agent de réticulation, et ledit agent de réticulation est sélectionné parmi le groupe constitué d'éther de butanediol-1,4 diglycidique, éther de poly(éthylène glycol) diglycidique, et éther d'éthylène glycol diglycidique.

4. Fil selon l'une quelconque des revendications 1 à 3, dans lequel une concentration d'hydroxyapatite de calcium dans ledit fil se situe entre 10 et 35 % massique.

5. Fil selon l'une quelconque des revendications 1 à 3, dans lequel une concentration d'hydroxyapatite de calcium dans ledit fil se situe entre 35 et 75 % massique.

6. Fil selon l'une quelconque des revendications précédentes, dans lequel ledit hydroxyapatite de calcium a une distribution de taille particulaire moyenne en poids entre 25 et 45 micromètres.

7. Procédé de fabrication d'un fil selon la revendication 1, le procédé comprenant :
la réticulation dans un milieu aqueux d'acide hyaluronique ou d'un sel de celui-ci en ajoutant un agent de réticulation et en augmentant le pH du milieu ;
la neutralisation dudit milieu aqueux ;
dans lequel de l'hydroxyapatite est ajouté à l'acide hyaluronique avant ou pendant la réticulation de l'acide hyaluronique,
procurant de la sorte une composition précurseur de fil ; formant ladite composition précurseur dans un fil humide ; et séchant ledit fil humide pour constituer un fil sec.

8. Procédé selon la revendication 7, comprenant l'ajout d'une base à un récipient à réaction qui a été préalablement chargé avec un acide hyaluronique et un agent de réticulation, pour former un mélange de réaction alcaline, permettant au mélange de réaction de commencer une réticulation, l'ajout et la dispersion de l'hydroxyapatite de calcium dans ledit récipient de réaction, permettant audit mélange de réaction dans ledit récipient de réaction de réaliser la réaction de réticulation, et la neutralisation dudit milieu aqueux en ajoutant un agent tampon pour amener la valeur de pH à une valeur essentiellement neutre.

9. Procédé selon l'une quelconque des revendications 7 ou 8, dans lequel ledit agent de réticulation est sélectionné parmi le groupe constitué d'éther de butanediol-1,4 diglycidique, éther de poly(éthylène glycol) diglycidique, et éther d'éthylène glycol diglycidique.

10. Procédé selon l'une quelconque des revendications 7 à 9, dans lequel une concentration dudit acide hyaluronique dans ledit milieu aqueux se situe entre 0,2 et 8 % massique.

11. Procédé selon l'une quelconque des revendications 7 à 10, dans lequel une concentration d'hydroxyapatite de calcium dans ledit milieu aqueux se situe entre 0,5 et 5 % massique.

12. Procédé selon l'une quelconque des revendications 7 à 10, dans lequel une concentration d'hydroxyapatite de calcium dans ledit milieu aqueux se situe entre 5 et 20 % massique.

13. Procédé selon l'une quelconque des revendications 7 à 12, dans lequel ledit hydroxyapatite de calcium a une distribution de taille particulaire moyenne en poids entre 25 et 45 micromètres.

14. Procédé selon l'une quelconque des revendications 7 à 13, dans lequel ladite formation comprend l'extrusion de ladite composition précurseur à travers un orifice dans un liquide de déshydratation ou sur une surface de séchage.

15. Procédé selon l'une quelconque des revendications 7 à 14, dans lequel le diamètre du fil est essentiellement uniforme, la variation du diamètre exprimée en tant qu'écart-type relatif de diamètre de mesures SEM prises le long de la longueur du fil étant inférieure à 20 %.
